(19) Europäisches Patentamt
European Patent Office
Office européen des brevets

(11) Veröffentlichungsnummer: **0 002 204**
**B1**

(12) **EUROPÄISCHE PATENTSCHRIFT**

(45) Veröffentlichungstag der Patentschrift: **02.09.81**

(51) Int. Cl.³: **C 07 C 153/017**, C 07 D 213/64, A 01 N 39/02

(21) Anmeldenummer: **78101372.7**

(22) Anmeldetag: **15.11.78**

(54) **Alpha-phenoxy-propionthiolsäuren und Salze, ihre Herstellung und Verwendung als Herbizide oder Zwischenprodukte.**

(30) Priorität: **24.11.77 CH 14398/77**

(43) Veröffentlichungstag der Anmeldung: **13.06.79 Patentblatt 79/12**

(45) Bekanntmachung des Hinweises auf die Patenterteilung: **02.09.81 Patentblatt 81/35**

(84) Benannte Vertragsstaaten: **BE CH DE FR GB NL**

(56) Entgegenhaltungen:
**DE-A-2 223 894**
**DE-A-2 383 165**
**DE-A-2 546 251**
**DE-A-2 637 911**

(73) Patentinhaber: **CIBA-GEIGY AG, Patentabteilung Postfach, CH-4002 Basel (CH)**

(72) Erfinder: **Böhner, Beat, Dr., Hügelweg 3, CH-4102 Binningen (CH)**
Erfinder: **Rohr, Otto, Dr., Kilbertweg 19, CH-4106 Therwil (CH)**
Erfinder: **Rempfler, Hermann, Dr., Allschwilerweg 67, CH-4102 Binningen (CH)**
Erfinder: **Pissiotas, Georg, Dr., Breslauer Strasse 8, D-7850 Lörrach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 204 B1

## Alpha-phenoxy-propionthiolsäuren und Salze, ihre Herstellung und Verwendung als Herbizide oder Zwischenprodukte

Vorliegende Erfindung betrifft neue herbizid wirksame, in para-Stellung substituierte α-Phenoxy-propionthiolsäuren und ihre Salze, Verfahren zu ihrer Herstellung, ferner herbizide Mittel, die diese neuen Verbindungen als Wirkstoffe enthalten, sowie Verfahren zur Bekämpfung von Unkräutern unter Verwendung der neuen Säuren und Salze oder der sie enthaltenden Mittel, sowie die Verwendung dieser Verbindungen als Zwischenprodukte für die Synthese weiterer herbizid wirksamen Derivate.

Die neuen Verbindungen entsprechen der Formel I

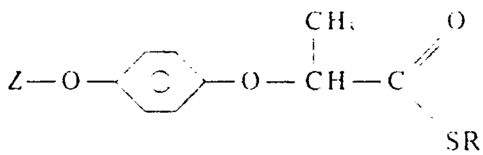
(I)

Darin bedeutet

R Wasserstoff, das Äquivalent eines Metallkations oder das Äquivalent eines Amin- oder Ammoniokations

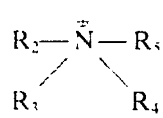

worin $R_2$ bis $R_5$ unabhängig voneinander je für Wasserstoff, Alkyl, substituiertes Alkyl, Cycloalkyl, Aralkyl oder Aryl stehen, oder zwei bzw. drei der Reste $R_2$ bis $R_5$ gemeinsam mit dem Stickstoffatom einen Heterocyclischen Ring bilden;

Z ist entweder ein substituierter Phenyl-(1)- oder ein substituierter Pyridyl-(2)-rest.

Als Substituenten eines Phenylrestes Z kommen einer oder zwei aus der Gruppe Halogen, $CF_3$, Cyano und Nitro in Betracht. Substituenten eines Pyridyl-(2)-restes Z sind Halogen und Trifluormethyl

Als Metallkationen R kommen Alkali- und Erdalkalimetallionen sowie die Ionen der Metalle Fe, Cu, Zn, Mn und Ni in erster Linie in Betracht. Mehrwertige Kationen der Wertigkeit n sind mit dem Äquivalent $\frac{1}{n}$ als Radikal R mit dem Rest der Säure liiert.

Von den vielen Aminen, die in protonierter Form als Kationen R in Frage kommen, seien folgende speziell erwähnt:

Ammoniak, Methylamin, Dimethylamin, Trimethylamin, Tributylamine, Triäthylamin, Tri-hydroxyäthylamin, Dimethylanilin, Diäthylanilin, N,N-Dimethylbenzylamin, Pyridin, Piperidin, Morpholin, Picoline.

Nachstehend sind bevorzugte substituierte Phenylreste Z angeführt:

4-Halogenphenyl
2,4-Dihalogenphenyl
4-Trifluormethylphenyl
2-Halogen-4-trifluormethylphenyl
4-Cyanophenyl
4-Halogen-2-cyanophenyl
4-Halogen-2-nitrophenyl

Von den substituierten Pyridyl-(2)-resten Z der Formel

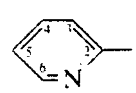

seien besonders hervorgehoben:

5-Halogen-pyridyl-(2)
3,5-Dihalogen-pyridyl-(2)
5-Trifluormethyl-pyridyl-(2)

Unter den Halogensubstituenten sind Brom und insbesondere Chlor bevorzugt.

Außer dem direkten Einsatz der neuen para-substituierten α-Phenoxy-propionthiolsäuren und ihrer Salze der Formel I als Herbizide, die aktiver sind als die entsprechenden Propionsäuren (Sauerstoffsäuren), können die Säuren der Formel I und Salze davon auch als Zwischenprodukte zur Synthese von anderen hochwirksamen Herbiziden, nämlich entsprechenden Thiolsäurederivaten wie Estern, Amiden etc. verwendet werden.

Ein Verfahren zur Herstellung der neuen substituierten α-Phenoxy-propionthiolsäuren und ihrer Salze der Formel I ist dadurch gekennzeichnet, daß man ein α-Phenoxy-propionsäurehalogenid der Formel II

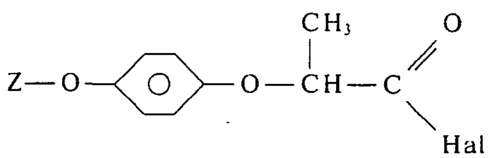

(II)

worin Z die unter Formel I angegebene Bedeutung hat und Hal ein Halogentaom, insbesondere Chlor darstellt, in Gegenwart eines basischen Säureakzeptors mit Schwefelwasserstoff oder einem seiner Salze umsetzt und aus dem Reaktionsgemisch das entstandene Salz der Thiolsäure oder durch Ansäuern die Thiolsäure selbst isoliert und diese gegebenenfalls in ein Salz überführt.

Die Umsetzung wird vorzugsweise in einem gegenüber den Reaktionskomponenten inerten Lösungsmittel durchgeführt. Als Lösungsmittel kommen solche aus den verschiedensten Stoffklassen in Frage, wie aliphate und aromatische Kohlenwasserstoffe und vorzugsweise polare organische Lösungsmittel, wie Alkohole, Äther, Ketone, Amide, stabile Ester, z. B. Methyläthylketon, Dimethoxyäthan, Dimethylformamid, Dimethylsulfoxid, Tetrahydrofuran. Man kann als Lösungsmittel aber auch eine organische Base (Amin) wie Pyridin verwenden, welches dann zugleich den Säureakzeptor darstellt und dabei selbstverständlich das entsprechende Salz der gebildeten Thiolpropionsäure schon im Reaktionsgemisch entsteht.

Als basischer Säureakzeptor können wässerige Alkalimetallhydroxyde, wie KOH und NaOH sowie weitere übliche basische Stoffe, wie Ammoniak, Karbonate ($K_2CO_3$, $NaHCO_3$), Alkoholate ($NaOCH_3$ und Kalium-tert.butylat), aber auch organische Basen wie Triäthylamin etc. verwendet werden. Dient eine organische Base (z. B. Pyridin) als Lösungsmittel, so erfüllt sie zugleich die Funktion des Säureakzeptors.

Anstelle von gasförmigem Schwefelwasserstoff, mit welchem gewisse Lösungsmittel vor der Umsetzung auch gesättigt werden können, kann man auch ein Sulfid oder Hydrogensulfid, wie z. B. $Na_2S$ oder NaHS verwenden. Zum Ansäuern verwendet man in der Regel eine Mineralsäure, wie konz. Salzsäure und stellt auf einen pH-Wert von etwa 1.

Die Ausgangsstoffe der Formel II, insbesondere die α-Phenoxy-propionsäurechloride lassen sich am besten aus den entsprechenden freien Propionsäuren durch Umsetzung mit Thionylchlorid $SOCl_2$ in bekannter Weise herstellen. Die freien kernsubstituierten α-Phenoxy-propionsäuren sind bekannte Verbindungen, die unter anderem in den DE-OS 2 223 894, 2 531 643, 2 546 251, 2 433 067 und 2 652 384 beschrieben worden sind.

Die Herstellung der Thiolsäuren der Formel I läuft im Prinzip meistens auf den Ersatz der Hydroxylgruppe in Sauerstoffsäuren durch die HS-Gruppe hinaus, wozu weitere Verfahrensvarianten möglich sind, die in »Houben–Weyl«, Band VIII, Seiten 480 und 481 (1952) beschrieben sind. Es handelt sich dabei um eine Acylierung von Schwefelwasserstoff.

So kann man auch die Säureanhydride in Gegenwart einer Base mit gasförmigen $H_2S$ umsetzen oder Säurechloride mit $H_2S$ in Gegenwart eines Aluminiumchloridkatalysators reagieren lassen. Ferner ist bei araliphatischen Säurechloriden auch deren Umsetzung mit kalter alkoholischer Kaliumsulfhydratlösung zur Darstellung von Thiolsäuren brauchbar.

Schließlich kann man eine Carbothiolgruppe

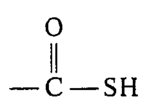

durch Einwirkenlassen von Kohlensulfoxid COS auf Grignard-Verbindungen des Typs $R_1-Mg-Hal$ direkt einführen, wobei neben Carbinolen die Thiolsäuren

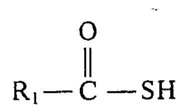

entstehen.

Die nachfolgenden Beispiele veranschaulichen ein erfindungsgemäßes Herstellungsverfahren für eine neue Thiolsäure und eines ihrer Salze. Weitere in entsprechender Weise hergestellte Thiolsäuren und deren Salze sind in der anschließenden Tabelle aufgeführt.

## Beispiel 1

34,5 g (0,1 Mol) α-[4-(4'-Trifluormethyl-phenoxy)-phenoxy]-propionsäurechlorid werden einem Gemisch von 17,9 g KOH (85%ig) in 8,9 ml Wasser und 150 ml Dimethoxyäthan zugetropft, das vorher bei 10 – 15° C unter starkem Rühren mit $H_2S$ gesättigt worden war. Während des Zutropfens hält man die Temperatur mit einem Eisbad bei 10° C. Anschließend läßt man 30 Min. bei Raumtemperatur ausrühren und gießt das Reaktionsgemisch auf 500 ml Wasser. Die trübe, braune Lösung wird mit konz. Salzsäure auf pH 1 gestellt. Dabei fällt ein braunes Öl aus, das in Methylenchlorid aufgenommen wird. Die organische Phase wird direkt auf eine kleine Kieselgel-Säule gegeben und mit Methylenchlorid durchgespült. Nach dem Eindampfen der hellgelben Lösung erhält man 34,0 g (99,4%) α-[4-(4'-Trifluormethylphenoxy)-phenoxy]-propion-thiolsäure als orangefarbenes, klares Öl mit einem n = 1,5421.

## Beispiel 2

13,7 g (0,04 Mol) der gemäß Beispiel 1 erhaltenen Thiolsäure werden in 20 ml Äther gelöst und mit 3,2 g (0,0405 Mol) Pyridin versetzt. Das Reaktionsgemisch wird für zwei Stunden auf 40° C erwärmt und anschließend eingedampft. Das orange, klare Öl kristallisiert beim Zerreiben mit Hexan gelb aus. Nach dem Abfiltrieren und Trocknen bei 40° C am Vakuum erhält man 6,5 g des Pyridiniumsalzes der α-[4-(4'-Trifluormethylphenoxy)-phenoxy]-propionthiolsäure in Form gelber Kristalle vom Schmelzpunkt 82 – 83° C.

In analoger Weise wurden folgende Verbindungen erhalten:

| Verbindung Nr. | Z | R | Physikalische Konstante n oder F |
|---|---|---|---|
| 1 | 4-Trifluormethylphenyl | H | n = 1,5421 |
| 2 | 4-Chlorphenyl | H | n = 1,5838 |
| 3 | 2,4-Dichlorphenyl | H | |
| 4 | 2-Chlor-4-trifluormethyl-phenyl | H | n = 1,5520 |
| 5 | 3,5-Dichlor-pyridyl-(2) | H | n = 1,5787 |
| 6 | 4-Trifluormethylphenyl | 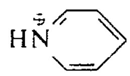 | F = 82–83° |
| 7 | 4-Trifluormethylphenyl | 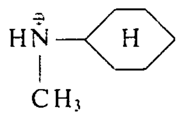 | F = 114–116° |
| 8 | 4-Trifluormethylphenyl | 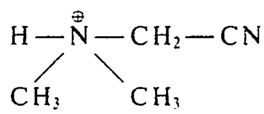 | n = 1,5282 |
| 9 | 3,5-Dichlor-pyridyl-(2) | 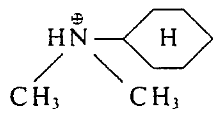 | n = 1,5881 |
| 10 | 2-Nitro-4-chlorphenyl | H | |
| 11 | 5-Chlor-pyridyl-(2) | H | |
| 12 | 2-Chlor-4-bromphenyl | H | |
| 13 | 5-Trifluormethyl-pyridyl-(2) | H | |
| 14 | 2-Cyano-4-chlorphenyl | H | |
| 15 | 4-Cyanophenyl | H | |
| 16 | 2,4-Dichlorphenyl | Na | Smp. >320° |
| 17 | 4-Trifluormethyl-phenyl | K | Smp. >320° |

Neben der Verwendbarkeit als herbizide Wirkstoffe sind die neuen Thiolsäuren und Salze der Formel I auch wichtige Zwischenprodukte für die Herstellung entsprechender Propionthiolsäureester mit herbizider Wirkung welche zum Teil bekannt und beispielsweise in den DE-OS 2 223 894 und 2 531 643 beschrieben, aber bisher auf anderem Wege hergestellt worden sind.

Mit den erfindungsgemäßen Propionthiolsäuren und ihren Salzen ist eine neue Gruppe von α-[4-(Phenoxy)-phenoxy]- und α-[4-(Pyridyloxy)-phenoxy]-thiolcarbonsäure-Derivaten erschlossen worden, die bei relativ niederen Aufwandmengen eine starke herbizide Wirkung zeigen und/oder das Pflanzenwachstum in anderer, für die Landwirtschaft nützlicher Weise zu regulieren vermögen.

Die neuen Wirkstoffe der Formel I sind als Herbizide im Vorauflauf-, als insbesondere auch im Nachauflauf-Verfahren brauchbar. Ihre Wirkung richtet sich vor allem gegen monocotyle Unkräuter, doch können sie auch zur Wuchshemmung bei dikotylen Pflanzen eingesetzt werden.

Die Wirkstoffe der Formel I sind wenig giftig für Warmblüter; die Aufwandmengen liegen vorteilhaft zwischen 0,1 und 5 kg Wirkstoff pro Hektar.

Die vorliegende Erfindung betrifft auch herbizide und pflanzenwachstumsregulierende Mittel, welche eine Verbindung der Formel I als aktive Komponente enthalten. Solche Mittel können in üblicher Weise als feste Formulierungen (Stäubemittel, Streumittel, Granulate) als in Wasser dispergierbare Konzentrate (Spritzpulver, Emulsionen, Emulsionskonzentrate und Pasten) oder als Lösungen vorliegen und werden nach bekannten Techniken mit entsprechenden Zuschlag- und Trägerstoffen formuliert.

**Patentansprüche**

1. Neue α-Phenoxy-propionthiolsäuren und deren Salze der Formel I

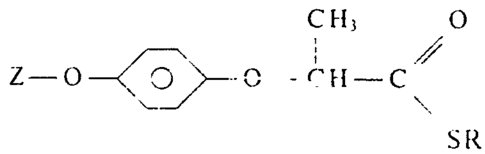 (I)

worin

R das Wasserstoffion, das Äquivalent eines Metallkations oder das Äquivalent eines Amin- oder Ammoniokations

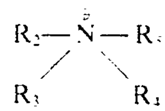

darstellt, worin $R_2$ bis $R_5$ unabhängig voneinander je für Wasserstoff, Alkyl, substituiertes Alkyl, Cycloalkyl, Aralkyl oder Aryl stehen oder zwei bzw. drei der Reste $R_2$ bis $R_5$ gemeinsam mit dem Stickstoffatom einen gesättigten oder ungesättigten heterocyclischen Ring bilden, und

Z entweder ein substituierter Phenyl-(1)- oder ein substituierter Pyridyl-(2)-Rest ist.

2. Phenoxypropionthiolsäuren und deren Salze gemäß Anspruch 1, dadurch gekennzeichnet, daß der Phenylrest Z durch einen oder zwei Substituenten aus der Gruppe Halogen, $CF_3$, Cyano und Nitro substituiert ist.

3. Phenoxypropionthiolsäuren und deren Salze gemäß Anspruch 1, dadurch gekennzeichnet, daß ein Pyridyl-(2)-Rest Z einen oder zwei Substituenten aus der Gruppe Halogen und Trifluormethyl($CF_3$) trägt.

4. Phenoxypropionthiolsäuren und deren Salze gemäß Anspruch 2, dadurch gekennzeichnet, daß ein Substituent in 4-Stellung, ein eventueller weiterer Substituent in 2-Stellung des Phenylrestes steht.

5. Phenoxypropionthiolsäuren und deren Salze gemäß Anspruch 3, dadurch gekennzeichnet, daß ein Substituent in 5-Stellung und ein eventueller weiterer Substituent in 3-Stellung des Pyridylrestes steht.

6. α-[4-(4'-Trifluoromethylphenoxy)-phenoxy]-propionthiolsäure und deren Pyridiniumsalz gemäß Anspruch 1.

7. Verfahren zur Herstellung der α-Phenoxy-propion-thiolsäuren und ihrer Salze von der Formel I des Anspruchs 1, dadurch gekennzeichnet, daß man ein α-Phenoxy-propionsäurehalogenid der Formel II

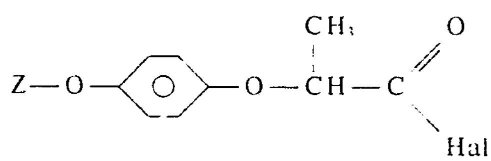 (II)

worin Z wie unter Formel I definiert ist und Hal ein Halogenatom, insbesondere Chlor darstellt, in Gegenwart eines basischen Säureakzeptors mit Schwefelwasserstoff oder einem seiner Salze umsetzt und aus dem Reaktionsgemisch das entstandene Salz der Thiolsäure oder durch Ansäuern die Thiolsäure selbst isoliert und diese gegebenenfalls in ein Salz überführt.

8. Herbizides Mittel, dadurch gekennzeichnet, daß es als wirksame Komponente mindestens eine α-Phenoxy-propionthiolsäure oder eines ihrer Salze der Formel I des Anspruchs 1 enthält.

9. Die Verwendung der α-Phenoxy-propionthiolsäuren und ihre Salze der Formel I, Anspruch 1 als Herbizide.

**Claims**

1. An α-phenoxy-thiolpropionic acid or a salt thereof of the formula I

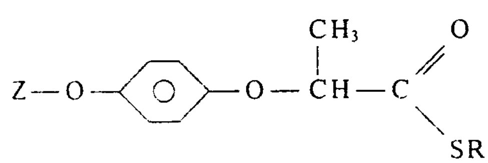 (I)

wherein R is the hydrogen ion, the equivalent of a metal cation or the equivalent of an amine or ammonium cation

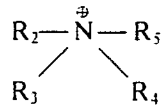

wherein each of $R_2$ to $R_5$ independently is hydrogen, alkyl, substituted alkyl, cycloalkyl, aralkyl or aryl, or two or three of the symbols $R_2$ to $R_5$, together with the nitrogen atom to which they are attached, form a saturated or unsaturated heterocyclic ring, and Z is either a substituted phenyl-(1) radical or a substituted pyridyl-(2) radical.

2. A phenoxythiolpropionic acid or a salt thereof according to claim 1, characterised in that a phenyl radical Z is substituted by one or two members selected from the group consisting of halogen, $CF_3$, cyano and nitro.

3. A phenoxythiolpropionic acid or a salt thereof according to claim 1, wherein a pyridyl-(2) radical Z is substituted by one or two members selected from the group consisting of halogen and trifluoromethyl ($CF_3$).

4. A phenoxythiolpropionic acid or a salt thereof according to claim 2, characterised in that one substituent is in the 4-position, and a possible further substituent is in the 2-position, of the phenyl radical.

5. A phenoxythiolpropionic acid or a salt thereof according to claim 3, characterised in that one substituent is in the 5-position, and a possible further substituent is in the 3-position, of the pyridyl radical.

6. α-[4-(4'-Trifluoromethylphenoxy)-phenoxy]thiolpropionic acid or the pyridinium salt thereof according to claim 1.

7. A process for the production of an α-phenoxy-thiolpropionic acid or a salt thereof of the formula I of claim 1, characterised in that an α-phenoxypropionic acid halide of the formula II

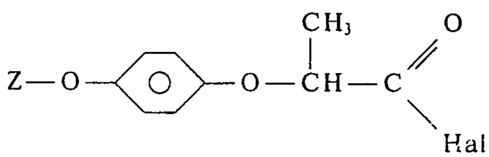

(II)

wherein Z is as defined in formula I and Hal is halogen atom, especially a chlorine atom, is reacted in the presence of a basic acid acceptor, with hydrogen sulfide or with a salt thereof, and the resultant salt of thiolic acid or, by acidification, the thiolic acid itself, is isolated from the reaction mixture and, if desired, this latter is converted into a salt.

8. A herbicidal composition characterised in that it contains, as active component, at least one α-phenoxy-thiolpropionic acid or a salt thereof of the formula I of claim 1.

9. The use of an α-phenoxythiolpropionic acid or a salt thereof of the formula I according to claim 1 as herbicide.

**Revendications**

1. Nouveaux acides α-phénoxy-propanethioïques et leurs sels de formule I

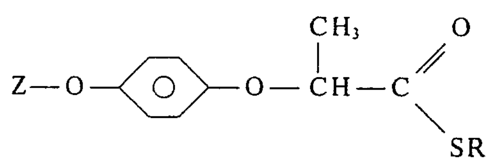

(I)

où R représente un hydrogène, l'équivalent d'un cation métallique ou l'équivalent d'un cation amine ou ammonium

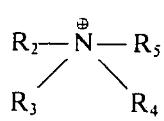

où $R_2$ à $R_5$ représentent indépendamment l'un de l'autre, chacun, un hydrogène, un alcoyle, un alcoyle substitué, un cycloalcoyle, un aralcoyle ou un aryle, ou bien où 2 ou 3 des radicaux $R_2$ à $R_5$ forment, avec

l'atome d'azote, un noyau hétérocyclique saturé ou insaturé, et Z est soit un radical phényl-(1) substitué, soit un radical pyridyl-(2) substitué.

2. Acides phénoxypropanethioïques et leurs sels selon la revendication 1, caractérisés en ce que le radical phényle Z est substitué par un ou deux substituants du groupe formé par les halogènes, $CF_3$, cyano et nitro.

3. Acides phénoxypropanethioïques et leurs sels selon la revendication 1, caractérisés en ce qu'un radical pyridyl-(2) Z porte un ou deux substituants du groupe formé par les halogènes et le trifluorométhyle ($CF_3$).

4. Acides phénoxypropanethioïques et leurs sels selon la revendication 2, caractérisés en ce qu'un substituant est en position 4, et un autre substituant éventuel en position 2 du radical phényle.

5. Acides phénoxypropanethioïques et leurs sels selon la revendication 3, caractérisés en ce qu'un substituant est en position 5 et un autre substituant éventuel en position 3 du radical pyridyle.

6. Acide α-[4-(4'-trifluorométhylphénoxy)-phénoxy]-propanethioïque et son sel de pyridinium selon la revendication 1.

7. Procédé de préparation des acides α-phénoxy-propanethioïques et de leurs sels de formule I selon la revendication 1, caractérisé en ce qu'on fait réagir un halogénure d'acide α-phénoxy-propionique de formule II

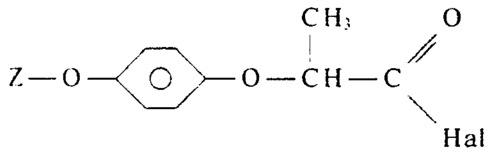

(II)

où Z a la signification donnée sous la formule I et où Hal représente un atome d'halogène, en particulier de chlore, en présence d'un accepteur d'acide basique avec de l'acide sulfhydrique ou un de ses sels et en ce qu'on isole du mélange réactionnel le sel de l'acide thioïque apparu ou par acidification l'acide thioïque lui-même et en ce qu'on transforme éventuellement celuici en un sel.

8. Agent herbicide, caractérisé en ce qu'il contient comme composant efficace au moins un acide α-phénoxypropanethioïque ou un de ses sels de formule I selon la revendication 1.

9. Application des acides α-phénoxy-propanethioïques et de leurs sels de formule I selon la revendication 1 comme herbicides.